# EUROPEAN PATENT APPLICATION

(11) **EP 4 786 570 A1**
(43) Date of publication of application: **05.08.2026**
(21) Application number: 24872105.2
(22) Date of filing: 20.09.2024
(51) Int. Cl.: C12M 1/00, B01F 23/50, B01F 35/71, B01F 101/44

(54) **POWDER SUPPLY DEVICE**

(30) Priority: 28.09.2023 JP 2023167583
(71) Applicant: FUJIFILM Corporation, Tokyo 106-8620 (JP)
(72) Inventor: HASEGAWA, Masataka, Tokyo 106-8620 (JP); INABA, Tatsuya, Tokyo 106-8620 (JP); YAMAMOTO, Takashi, Tokyo 106-8620 (JP)
(74) Representative: Klunker IP Patentanwälte PartG mbB
(86) International application number: PCT/JP2024/033735
(87) International publication number: WO 2025/070314

(57) **Abstract**

A powder supply device that continuously supplies a powder in order to continuously prepare a bioprocess solution having a set concentration in a mixing container, the powder supply device including a first container that stores the powder, the first container being connected to the mixing container via a feeder and supplying the powder to the feeder, and a second container that stores the powder, the second container being connected to the first container and supplying the powder to the first container.

## Description

### BACKGROUND OF THE INVENTION

### 1. Field of the Invention

A technology of the present disclosure relates to a powder supply device.

### 2. Description of the Related Art

In a bioprocess in which Chinese hamster ovary cells into which an antibody gene has been incorporated are cultured to obtain an antibody pharmaceutical, various bioprocess solutions are required. WO2021/123248A discloses a method of continuously preparing a bioprocess solution having a set concentration by continuously supplying a solvent and a powder to a mixing container and mixing the solvent and the powder in the mixing container. The mixing container is connected to the bioprocess container, and the bioprocess solution is continuously supplied from the mixing container to the bioprocess container. The solvent is, for example, purified water such as pure water, the powder is, for example, a powder culture medium, and the bioprocess solution is, for example, a culture medium (culture solution). In addition, the bioprocess container is, for example, a culture tank.

### SUMMARY OF THE INVENTION

For example, in continuous preparation of the bioprocess solution over a long period of time such as one month, it is essential to stably supply the powder to the mixing container. However, in WO2021/123248A, no measures are taken for the stable supply of the powder to the mixing container.

One embodiment according to the disclosed technology provides a powder supply device that can contribute to a stable supply of the powder to the mixing container.

A powder supply device according to the present disclosure is A powder supply device that continuously supplies a powder in order to continuously prepare a bioprocess solution having a set concentration in a mixing container, the powder supply device comprising a first container that stores the powder, the first container being connected to the mixing container via a feeder and supplying the powder to the feeder, and a second container that stores the powder, the second container being connected to the first container and supplying the powder to the first container.

It is preferable that the second container is exchangeably connected to the first container.

It is preferable that the first container expands and contracts.

It is preferable that the first container is sealed.

It is preferable that a part of the first container expands and contracts.

It is preferable that the second container expands and contracts.

It is preferable that the second container is sealed.

It is preferable that a part of the second container expands and contracts.

It is preferable that the first container has a first ventilation port.

It is preferable that a humidity-controlled air is supplied to an inside of the first container from the first ventilation port by a humidity control mechanism.

It is preferable that the first container does not expand and contract.

It is preferable that the second container has a second ventilation port.

It is preferable that the second container does not expand and contract.

It is preferable that the first container and the second container expand and contract.

It is preferable that the first container expands and contracts, and the second container has a second ventilation port.

It is preferable that the first container has a first ventilation port, the second container expands and contracts, and a humidity-controlled air is supplied to an inside of the first container from the first ventilation port by a humidity control mechanism.

It is preferable that the first container has a first ventilation port, the second container has a second ventilation port, and a humidity-controlled air is supplied to an inside of the first container from the first ventilation port by a humidity control mechanism.

It is preferable that a solvent that is mixed with the powder in the mixing container is purified water, the powder being a powder culture medium, and the bioprocess solution is a culture medium.

It is preferable that the culture medium is continuously supplied from the mixing container to a culture tank.

It is preferable that the first container and the second container are used once and then discarded.

According to the disclosed technology, it is possible to provide a powder supply device that can contribute to a stable supply of the powder to the mixing container.

### BRIEF DESCRIPTION OF THE DRAWINGS

FIG. 1 is a diagram showing a culture medium preparation device and a culture tank.
FIG. 2 is a diagram showing a powdered culture medium supply device.
FIG. 3 is a diagram showing a processing procedure of the powdered culture medium supply device.
FIG. 4 is a diagram showing a first container and a second container in which a part expands and contracts.
FIG. 5 is a diagram showing another example of the first container in which a part expands and contracts.
FIG. 6 is a diagram showing a powdered culture medium supply device of a second embodiment.
FIG. 7 is a diagram showing a powdered culture medium supply device of a third embodiment.
FIG. 8 is a diagram showing another example of the humidity control mechanism.
FIG. 9 is a diagram showing still another example of the humidity control mechanism.
FIG. 10 is a diagram showing a powdered culture medium supply device of a fourth embodiment.
FIG. 11 is a table summarizing a combination that can be considered in the first embodiment in which both the first container and the second container expand and contract and an evaluation of the combination.
FIG. 12 is a table summarizing a combination that can be considered in the second embodiment in which the first container expands and contracts and the second container does not expand and contract and an evaluation of the combination.
FIG. 13 is a table summarizing a combination that can be considered in the third embodiment in which the first container does not expand and contract and the second container expands and contracts and an evaluation of the combination.
FIG. 14 is a table summarizing a combination that can be considered in the fourth embodiment in which both the first container and the second container do not expand and contract and an evaluation of the combination.

### DESCRIPTION OF THE PREFERRED EMBODIMENTS

### [First Embodiment]

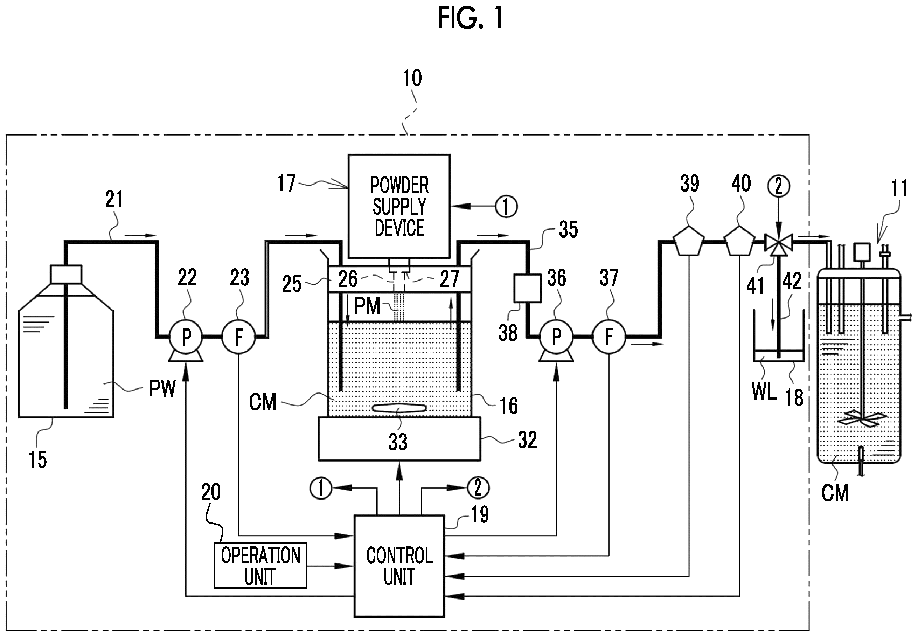

As shown in FIG. 1 as an example, the culture medium preparation device 10 prepares a culture medium CM. The culture medium preparation device 10 is connected to the culture tank 11, and continuously supplies the prepared culture medium CM to the culture tank 11. The culture medium CM is an example of a "bioprocess solution" according to the technology of the present disclosure. It is noted that in FIG. 1, an example in which the culture medium preparation device 10 is directly connected to the culture tank 11 has been described, but the present invention is not limited thereto. A tank for temporarily storing the culture medium CM may be provided between the culture medium preparation device 10 and the culture tank 11.

For example, Chinese hamster ovary cells into which an antibody gene has been incorporated are seeded in the culture tank 11. In the culture tank 11, Chinese hamster ovary cells are cultured in the culture medium CM. As described above, since the culture medium CM is continuously supplied to the culture tank 11 from the culture medium preparation device 10, the culture of the Chinese hamster ovary cells performed in the culture tank 11 is perfusion culture. The culture tank 11 is connected to a purification device (not shown). The purification device purifies an antibody produced by Chinese hamster ovary cells in the culture process, and yields the antibody as an active pharmaceutical ingredient of an antibody pharmaceutical.

The culture medium preparation device 10 includes a pure water storage tank 15, a mixing container 16, a powdered culture medium supply device 17, a waste liquid recovery tank 18, a control unit 19, an operation unit 20, and the like.

The pure water storage tank 15 stores pure water PW. The pure water PW is an example of a "solvent" and "purified water" according to the disclosed technology.

One end of the pure water supply line 21 is connected to the pure water storage tank 15. The other end of the pure water supply line 21 is connected to the mixing container 16. A pure water supply pump 22 and a pure water flowmeter 23 are provided in the pure water supply line 21. The pure water supply pump 22 is driven and controlled by the control unit 19. In a case where the pure water supply pump 22 is driven, the pure water PW in the pure water storage tank 15 is supplied to the mixing container 16 through the pure water supply line 21. The pure water flowmeter 23 is disposed downstream of the pure water supply pump 22, and measures a flow rate of the pure water PW passing through the pure water supply line 21. The pure water flowmeter 23 outputs the measured flow rate to the control unit 19. The control unit 19 controls the driving of the pure water supply pump 22 such that the flow rate of the pure water flowmeter 23 is a set amount.

The mixing container 16 has, for example, a wide cylindrical shape and is a container for mixing the pure water PW and a powder culture medium PM to generate the culture medium CM having a set concentration. The upper portion of the mixing container 16 is sealed with a lid 25. A through hole 26 is formed in a center portion of the lid 25. A discharge port 27 for the powdered culture medium PM of the powdered culture medium supply device 17 is aseptically connected to an upper end part of the through hole 26. As a result, the mixing container 16 and the powdered culture medium supply device 17 are aseptically connected. The powdered culture medium PM discharged from the discharge port 27 is supplied to the mixing container 16 through the through hole 26. The powder culture medium PM is an example of a "powder" according to the disclosed technology.

The powdered culture medium supply device 17 continuously discharges the set amount of the powder culture medium PM from the discharge port 27 under the driving control of the control unit 19. The powdered culture medium supply device 17 is an example of a "powder supply device" according to the disclosed technology.

The mixing container 16 is installed on the stirrer 32. A stirring bar 33 is put in the mixing container 16. The stirring bar 33 has an elongated cocoon-like shape in which both end portions are rounded and a central portion is slightly swollen. A magnet is built in the stirring bar 33. The stirrer 32 is driven and controlled by the control unit 19. The stirrer 32 generates a magnetic force for rotating the stirring bar 33. The stirrer 32 rotates the stirring bar 33 at a set rotation speed to mix the pure water PW and the powdered culture medium PM to obtain a culture medium CM.

One end of the culture medium supply line 35 is connected to the mixing container 16. The other end of the culture medium supply line 35 is connected to the culture tank 11. A culture medium supply pump 36 and a culture medium flowmeter 37 are provided in the culture medium supply line 35. The culture medium supply pump 36 is driven and controlled by the control unit 19. In a case where the culture medium supply pump 36 is driven, the culture medium CM in the mixing container 16 is supplied to the culture tank 11 through the culture medium supply line 35. The culture medium flowmeter 37 is disposed downstream of the culture medium supply pump 36, and measures the flow rate of the culture medium CM passing through the culture medium supply line 35. The culture medium flowmeter 37 outputs the measured flow rate to the control unit 19. The control unit 19 controls the driving of the culture medium supply pump 36 such that the flow rate of the culture medium flowmeter 37 is a set amount.

The flow rate of the pure water PW and the flow rate of the culture medium CM are set to values such that the amount of liquid in the mixing container 16 does not change from the beginning to the end. More specifically, the flow rate of the culture medium CM is set according to the combined amount of the flow rate of the pure water PW and the supply amount of the powdered culture medium PM. In practice, the weight of the mixing container 16 is measured with a weighing scale, and the flow rate of the pure water PW and the flow rate of the culture medium CM are set such that the measured weight is constant.

A filter 38 is disposed upstream of the culture medium supply pump 36 of the culture medium supply line 35. The filter 38 removes unnecessary substances in the culture medium CM. The unnecessary substance is, for example, an undissolved powdered culture medium PM, and the like.

A conductivity meter 39 and a hydrogen-ion exponent meter 40 are disposed downstream of the culture medium flowmeter 37 in the culture medium supply line 35. In addition, a waste liquid line 42 is connected to the downstream of the hydrogen-ion exponent meter 40 of the culture medium supply line 35 via a three-way valve 41. The three-way valve 41 is driven and controlled by the control unit 19. The waste liquid recovery tank 18 is connected to the waste liquid line 42. The waste liquid recovery tank 18 recovers the waste liquid WL which is the culture medium CM not having the set concentration or the culture medium CM having an abnormal value of the hydrogen-ion exponent.

The conductivity meter 39 measures the conductivity (unit: mS/cm) of the culture medium CM that passes through the culture medium supply line 35. The conductivity meter 39 outputs the measured conductivity to the control unit 19. The control unit 19 derives the concentration of the culture medium CM based on the conductivity from the conductivity meter 39 and determines whether or not the derived concentration is the set concentration. In a case where it is determined that the culture medium CM is not at the set concentration, the control unit 19 sets the flow passage of the three-way valve 41 to the waste liquid line 42 side, and discharges the waste liquid WL, which is the culture medium CM not having the set concentration, to the waste liquid recovery tank 18. On the other hand, in a case where it is determined that the culture medium CM has the set concentration, the control unit 19 sets the flow passage of the three-way valve 41 to the culture tank 11 side, and introduces the culture medium CM having the set concentration into the culture tank 11. In this way, only the culture medium CM having the set concentration is supplied to the culture tank 11. Accordingly, it is possible to prevent a problem such as the supply of the culture medium CM not having the set concentration to the culture tank 11 and the failure of the culture of the Chinese hamster ovary cells.

The hydrogen-ion exponent meter 40 measures the hydrogen-ion exponent of the culture medium CM that passes through the culture medium supply line 35. The hydrogen-ion exponent meter 40 outputs the measured hydrogen-ion exponent to the control unit 19. In a case where the hydrogen-ion exponent from the hydrogen-ion exponent meter 40 is an abnormal value, the control unit 19 sets the flow passage of the three-way valve 41 to the waste liquid line 42 side, and discharges the waste liquid WL, which is the culture medium CM having an abnormal value of the hydrogen-ion exponent, to the waste liquid recovery tank 18.

The control unit 19 includes, for example, a processor such as a central processing unit (CPU), a memory, and a storage, and comprehensively controls driving of each unit of the culture medium preparation device 10. The processor of the control unit 19 may be a programmable logic device (PLD) that is a processor of which a circuit configuration can be changed after manufacture, such as a field programmable gate array (FPGA), a dedicated electric circuit that is a processor having a circuit configuration dedicatedly designed to execute specific processing, such as an application specific integrated circuit (ASIC), or the like.

The operation unit 20 is, for example, a touch panel, and receives various operation instructions from an operator of the culture medium preparation device 10. The various operation instructions include an instruction to start the continuous supply of the pure water PW and the powder culture medium PM to the mixing container 16 and an instruction to forcibly stop the supply of the culture medium CM from the mixing container 16 to the culture tank 11.

The pure water storage tank 15, the mixing container 16, and the waste liquid recovery tank 18 are single-use. In addition, the pure water supply line 21, the culture medium supply line 35, the filter 38, the three-way valve 41, and the waste liquid line 42 are also single-use.

All flow passages from the pure water storage tank 15 to the culture tank 11 through the pure water supply line 21, the mixing container 16, and the culture medium supply line 35 are aseptically connected. In addition, the culture medium supply line 35, the three-way valve 41, and the waste liquid line 42 are also aseptically connected.

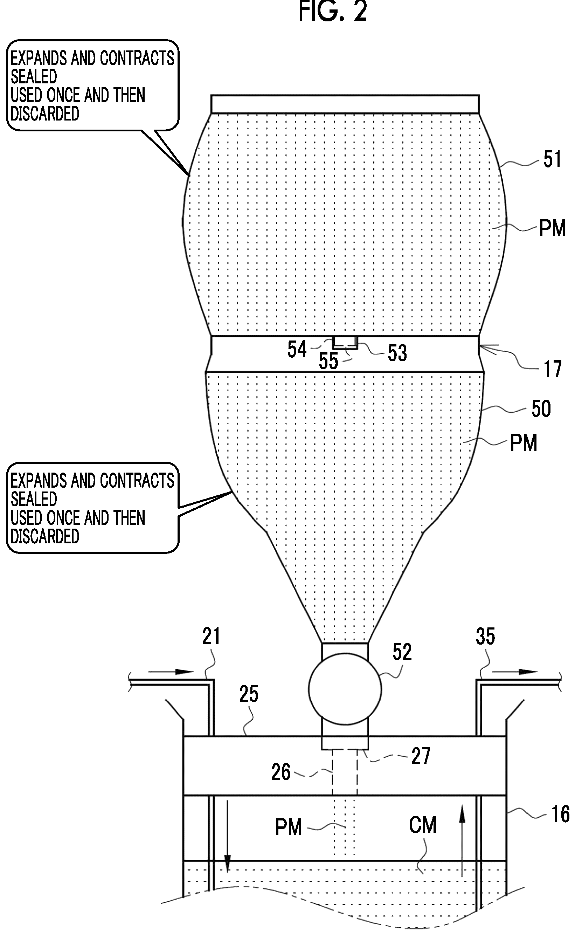

As shown in FIG. 2 as an example, the powdered culture medium supply device 17 comprises a first container 50, a second container 51, and a feeder 52. Both the first container 50 and the second container 51 store the powder culture medium PM. Capacities of the first container 50 and the second container 51, that is, the amounts of the powder culture medium PM stored in the first container 50 and the second container 51 are substantially the same. The first container 50 and the second container 51 are used by being suspended from a hook or the like.

The first container 50 and the second container 51 are aseptically closed bags formed of a soft resin that is suitable for United States Pharmacopeia (USP) Class 6, such as polyethylene, polyvinyl chloride, or an ethylene-vinyl acetate copolymer, for example. That is, both the first container 50 and the second container 51 expand and contract and are sealed. In addition, the first container 50 and the second container 51 are also single-use, like the mixing container 16 and the like. The term "expand and contract" means that the container is flexible and deforms in response to a change in pressure inside the container.

The first container 50 has a funnel shape, and the feeder 52 is attached to a lower portion having a narrowed diameter. The feeder 52 is connected to the discharge port 27 for discharging the powder culture medium PM to the mixing container 16. Therefore, it can be said that the first container 50 is connected to the mixing container 16 via the feeder 52. The feeder 52 may be attachably and detachably attached to the first container 50 or may be attached to the first container 50 in a non-attachable and detachable manner.

The first container 50 supplies the powder culture medium PM to the feeder 52. That is, the first container 50 plays a role of a so-called hopper. The feeder 52 continuously discharges the powder culture medium PM from the first container 50 to the mixing container 16 from the discharge port 27 under the control of the control unit 19. The feeder 52 is, for example, a screw feeder (also referred to as an auger feeder) that continuously discharges the set amount of the powder culture medium PM from the discharge port 27 by rotationally driving a helical screw with a motor. The feeder 52 is not limited to the example of the screw feeder, and may be a table feeder (also referred to as a circle feeder), a rotary feeder, a belt feeder, a vibration feeder, or the like. A stirrer for crushing a rathole generated by long-term use may be introduced into the first container 50. In addition, the first container 50 and the second container 51 may be subjected to a sliding treatment on an inner surface thereof to improve the dischargeability of the powder culture medium PM.

A first connecting portion 53 is provided on an upper portion of the first container 50 opposite to a lower portion on which the feeder 52 is attached. In addition, a second connecting portion 54 corresponding to the first connecting portion 53 is provided on a lower portion of the second container 51. The second container 51 is exchangeably connected to the first container 50 by the first connecting portion 53 and the second connecting portion 54.

The second connecting portion 54 is open in a case of being connected to the first connecting portion 53 and functions as a supply port for supplying the powder culture medium PM in the second container 51 to the first container 50. For example, a seal is attached to the second connecting portion 54, and the seal is broken by a blade formed in the first connecting portion 53, whereby the second connecting portion 54 is opened.

A valve 55 is provided in the first connecting portion 53. The control unit 19 opens and closes the valve 55 in response to the operation instruction of the operator input via the operation unit 20. The valve 55 is opened and closed to supply the powder culture medium PM of the second container 51 to the first container 50 or to stop the supply.

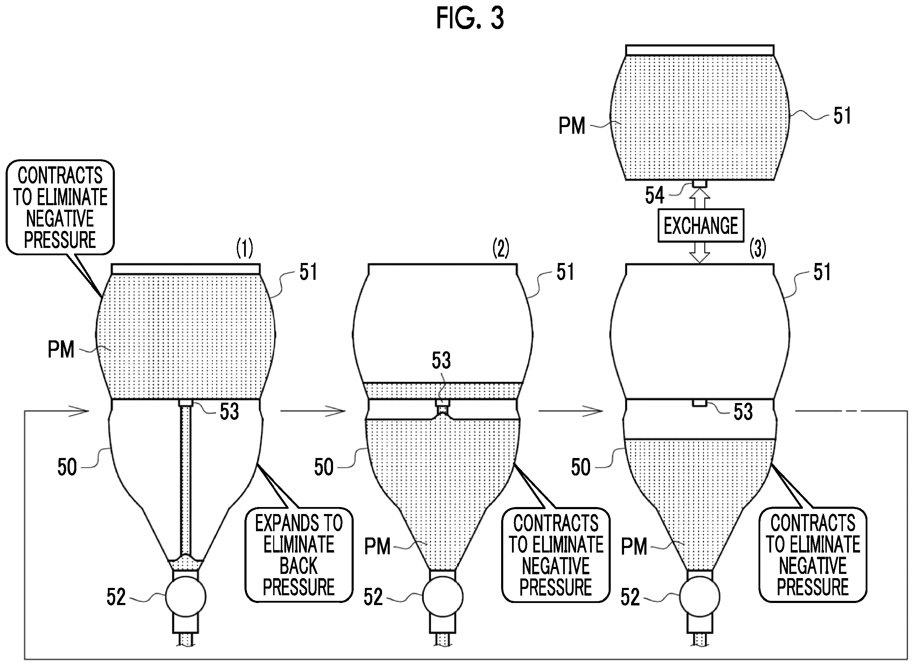

As shown in (1) of FIG. 3 as an example, in a case in which the amount of the powder culture medium PM stored in the first container 50 is equal to or less than the set amount and the operation instruction to open the valve 55 is input to the operation unit 20 by the operator, the valve 55 is opened under the control of the control unit 19. As a result, the powder culture medium PM is supplied from the second container 51 to the first container 50 via the first connecting portion 53 and the second connecting portion 54. The amount of the powder culture medium PM supplied from the second container 51 to the first container 50 per unit time exceeds the amount of the powder culture medium PM supplied from the first container 50 to the mixing container 16 per unit time. Therefore, the supply of the powder culture medium PM from the second container 51 to the first container 50 does not fall behind, and the powder culture medium PM in the first container 50 is not likely to be depleted.

As the powder culture medium PM is supplied from the second container 51 to the first container 50, a back pressure is generated in the first container 50, and a negative pressure is generated in the second container 51. Therefore, the supply of the powder culture medium PM from the second container 51 to the first container 50 is delayed as it is. However, since the first container 50 and the second container 51 expand and contract, the first container 50 expands to eliminate the back pressure, and the second container 51 contracts to eliminate the negative pressure. Therefore, the supply of the powder culture medium PM from the second container 51 to the first container 50 is not likely to be delayed due to the back pressure in the first container 50 and the negative pressure in the second container 51.

In addition, as shown in (2), in a case in which the powder culture medium PM is substantially completely supplied from the second container 51 to the first container 50, the first container 50 is in a negative pressure as the powder culture medium PM is supplied from the first container 50 to the mixing container 16 via the feeder 52. Therefore, the supply of the powder culture medium PM from the first container 50 to the mixing container 16 is delayed as it is. However, since the first container 50 expands and contracts, the first container 50 contracts to eliminate the negative pressure. Therefore, the supply of the powder culture medium PM from the first container 50 to the mixing container 16 is not likely to be delayed due to the negative pressure in the first container 50.

As shown in (3), in a case in which the supply of the powder culture medium PM from the second container 51 to the first container 50 is ended, the operation instruction to close the valve 55 is input to the operation unit 20 by the operator, and the valve 55 is closed under the control of the control unit 19. Then, the empty second container 51 is removed from the first container 50 by the operator, and a new second container 51 filled with the powder culture medium PM is attached to the first container 50. That is, the second container 51 is exchanged.

The powder culture medium PM has a property of solidifying due to moisture absorption. In a case in which the powder culture medium PM in the first container 50 solidifies due to moisture absorption, the supply of the powder culture medium PM from the first container 50 to the mixing container 16 is delayed. However, since the first container 50 is sealed, the powder culture medium PM in the first container 50 is not likely to solidify due to moisture absorption. Therefore, the supply of the powder culture medium PM from the first container 50 to the mixing container 16 is not likely to be delayed due to the solidification of the powder culture medium PM due to moisture absorption.

The processing shown in (1) to (3) is repeatedly performed as long as the culture in the culture tank 11 continues without stopping the entire operation of the culture medium preparation device 10 including the supply of the powder culture medium PM to the mixing container 16 by the feeder 52. The exchange of the second container 51 shown in (3) may be performed at any timing from the end of the supply of the powder culture medium PM from the second container 51 to the first container 50 until the amount of the powder culture medium PM stored in the first container 50 is equal to or less than the set amount.

As described above, the powdered culture medium supply device 17 is a device that continuously supplies the powder culture medium PM to the mixing container 16 in order to continuously prepare the culture medium CM having a set concentration, and comprises the first container 50 and the second container 51. Both the first container 50 and the second container 51 store the powder culture medium PM. The first container 50 is connected to the mixing container 16 via the feeder 52 and supplies the powder culture medium PM to the feeder 52. The second container 51 is connected to the first container 50 and supplies the powder culture medium PM to the first container 50. The addition of the second container 51 makes it possible to contribute to a stable supply of the powder culture medium PM to the mixing container 16 as compared with a case of only the first container 50.

As shown in (3) of FIG. 3, the second container 51 is exchangeably connected to the first container 50. Therefore, it is possible to greatly contribute to the stable supply of the powder culture medium PM to the mixing container 16. As a result, for example, the culture medium CM can be continuously prepared over a long period of time such as one month. As compared with a case in which the first container 50 is increased in capacity, such as setting the capacity of the first container 50 to be 50 times the capacity of the second container 51, the powdered culture medium supply device 17 can be reduced in size. In addition, as compared with a case in which the first container 50 is increased in capacity, the handling property of the powdered culture medium supply device 17 can be improved.

The supply of the powder culture medium PM from the second container 51 to the first container 50 is completed by exchanging the second container 51 and opening the valve 55. Therefore, the powder culture medium PM can be easily supplied from the second container 51 to the first container 50. Since the powder culture medium PM is not scattered, the surrounding environment of the powdered culture medium supply device 17 can be kept clean.

As shown in FIG. 2, the first container 50 and the second container 51 expand and contract. Therefore, the supply of the powder culture medium PM from the second container 51 to the first container 50 can be smoothly performed without being hindered by the back pressure in the first container 50 and the negative pressure in the second container 51. In addition, the supply of the powder culture medium PM from the first container 50 to the mixing container 16 can be smoothly performed without being hindered by the negative pressure in the first container 50.

As shown in FIG. 2, the first container 50 is sealed. Therefore, the powder culture medium PM in the first container 50 is not likely to solidify due to moisture absorption. Therefore, the supply of the powder culture medium PM from the first container 50 to the mixing container 16 can be smoothly performed without being hindered by the solidification of the powder culture medium PM in the first container 50.

As shown in FIG. 2, the second container 51 is also sealed. Therefore, the powder culture medium PM in the second container 51 is not likely to solidify due to moisture absorption. Therefore, the supply of the powder culture medium PM from the second container 51 to the first container 50 can be smoothly performed without being hindered by the solidification of the powder culture medium PM in the second container 51.

As shown in FIG. 1, the solvent that is mixed with the powder culture medium PM in the mixing container 16 is the pure water PW, the powder is the powder culture medium PM, and the bioprocess solution is the culture medium CM. Therefore, the culture medium CM indispensable for the culture can be stably prepared for a relatively long period of time.

In addition, the culture medium CM is continuously supplied from the mixing container 16 to the culture tank 11. Therefore, it is possible to employ perfusion culture, and it is possible to perform a relatively long-term culture close to a physiological environment.

As shown in FIG. 2, both the first container 50 and the second container 51 are used once and then discarded. Therefore, it is possible to suppress contamination due to repeated use.

### (Modification Example of First Embodiment)

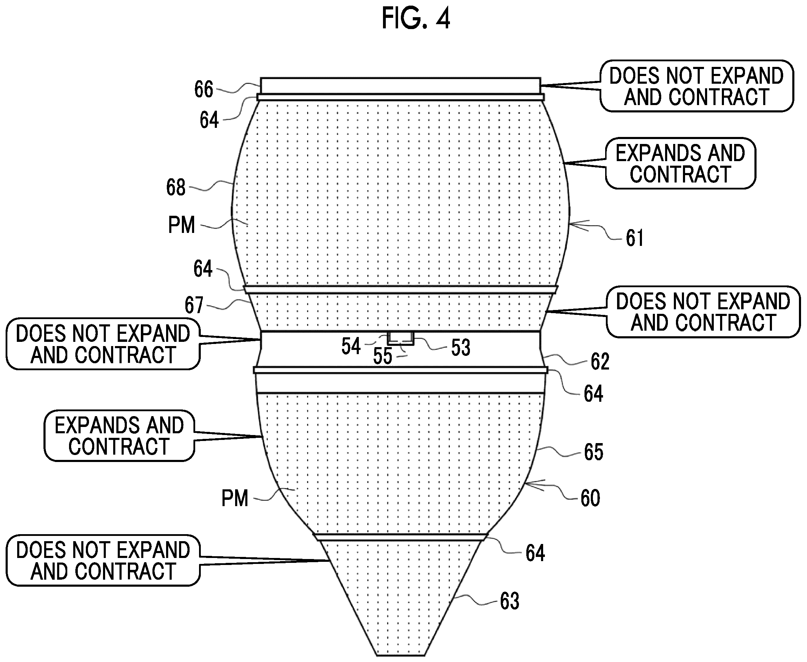

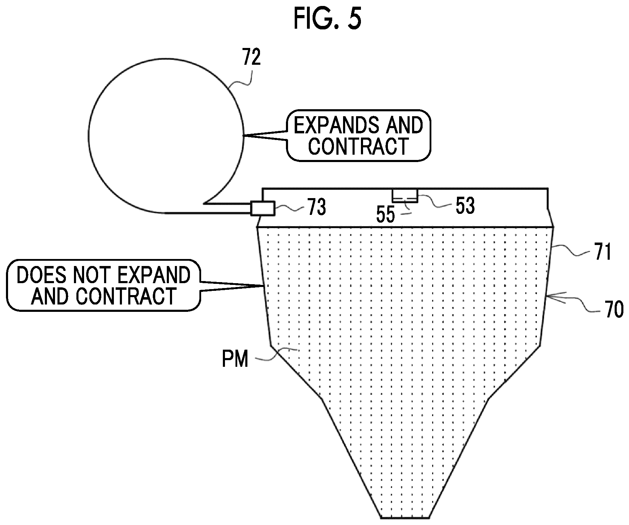

In FIG. 2, the first container 50 and the second container 51 that expand and contract as a whole are shown as an example, but the present disclosure is not limited thereto. As an example, as shown in FIG. 4, a first container 60 and a second container 61 in which a part expands and contracts may be used. The same reference numerals are given to portions common to the first container 50 and the second container 51, such as the first connecting portion 53 and the second connecting portion 54, and the description thereof will not be repeated. The same applies to FIGS. 5 to 10.

The first container 60 is composed of an upper portion 62 to which the second container 61 is connected, a lower portion 63 having a narrowed diameter to which the feeder 52 is connected, and a central portion 65 integrated with the upper portion 62 and the lower portion 63 by a sealing material 64. Both the upper portion 62 and the lower portion 63 are formed of, for example, a hard resin suitable for USP Class 6, such as polyethylene, polypropylene, or polycarbonate. On the other hand, the central portion 65 is formed of a soft resin suitable for USP Class 6. That is, the upper portion 62 and the lower portion 63 do not expand and contract, and only the central portion 65 expands and contracts.

The second container 61 is composed of an upper portion 66, a lower portion 67 connected to the first container 60, and a central portion 68 integrated with the upper portion 66 and the lower portion 67 by the sealing material 64. The upper portion 66 and the lower portion 67 are formed of a hard resin suitable for USP Class 6, like the upper portion 62 and the lower portion 63 of the first container 60. On the other hand, the central portion 68 is formed of a soft resin suitable for USP Class 6, like the central portion 65 of the first container 60. That is, the upper portion 66 and the lower portion 67 do not expand and contract, and only the central portion 68 expands and contracts. With such a configuration, the supply of the powder culture medium PM from the second container 61 to the first container 60 and the supply of the powder culture medium PM from the first container 60 to the mixing container 16 can be smoothly performed without being hindered by the back pressure and the negative pressure in the first container 60 and the negative pressure in the second container 61.

In a case in which the upper portion 62 to which the second container 61 is connected and the lower portion 63 to which the feeder 52 is attached do not expand and contract as in the first container 60, the installation stability of the second container 61 on the first container 60 and an installation stability of the first container 60 on the feeder 52 can be improved. In addition, in a case in which the lower portion 67 connected to the first container 60 does not expand and contract as in the second container 61, the installation stability of the second container 61 on the first container 60 can be improved.

In addition, as an example, a configuration shown in FIG. 5 may be adopted. In FIG. 5, the first container 70 is composed of a body portion 71 and a balloon 72. The balloon 72 is attached to a first ventilation port 73 provided on an upper portion of the body portion 71 where the powder culture medium PM is not stored, and is connected to the body portion 71 through the first ventilation port 73. The body portion 71 is formed of a hard resin suitable for USP Class 6. On the other hand, the balloon 72 is formed of a soft resin suitable for USP Class 6. That is, the body portion 71 does not expand and contract, and only the balloon 72 expands and contracts. With such a configuration, the supply of the powder culture medium PM from the second container 51 to the first container 70 and the supply of the powder culture medium PM from the first container 70 to the mixing container 16 can be smoothly performed without being hindered by the back pressure and the negative pressure in the first container 70. In FIG. 5, the first container 70 is shown as an example, but the same configuration may be adopted for the second container.

### [Second Embodiment]

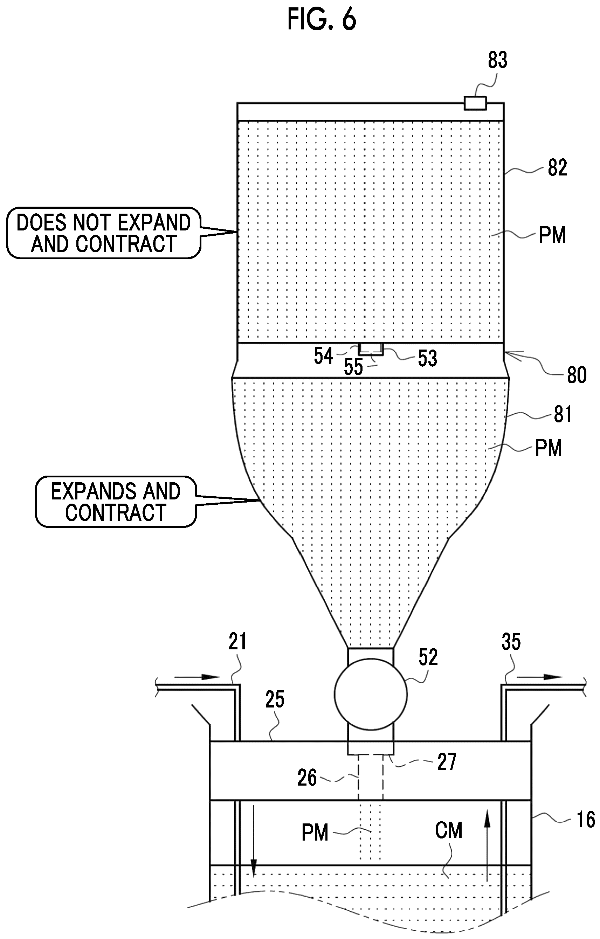

In the first embodiment, an aspect in which the first container 50 and the second container 51 expand and contract has been described as an example, but the present disclosure is not limited thereto. As a powdered culture medium supply device 80 shown in FIG. 6 as an example, an aspect in which a first container 81 is formed of a soft resin suitable for USP Class 6 and expands and contracts and a second container 82 is formed of a hard resin suitable for USP Class 6 and does not expand and contract may be adopted.

The second container 82 has a second ventilation port 83 on an upper portion where the powder culture medium PM is not stored. The second ventilation port 83 functions as an intake port that introduces outside air into the second container 82 in order to eliminate the negative pressure in the second container 82 generated in conjunction with the supply of the powder culture medium PM from the second container 82 to the first container 81.

A seal is attached to the second ventilation port 83. The seal is peeled off by the operator in a case in which the supply of the powder culture medium PM from the second container 82 to the first container 81 is started. A filter may be attached to the second ventilation port 83. As the filter, for example, an air vent (Aevent)-50 hydrophobic disposable filter manufactured by Merck KGaA can be used.

As described above, in the second embodiment, the first container 81 expands and contracts, and the second container 82 has the second ventilation port 83. With such a configuration, the supply of the powder culture medium PM from the second container 82 to the first container 81 and the supply of the powder culture medium PM from the first container 81 to the mixing container 16 can be smoothly performed without being hindered by the back pressure and the negative pressure in the first container 81 and the negative pressure in the second container 82. However, since the second container 82 needs to have the second ventilation port 83, the powdered culture medium supply device 80 of the second embodiment has a more complicated configuration than the powdered culture medium supply device 17 of the first embodiment.

The second container 82 does not expand and contract. Therefore, the second container 82 can be fixed to a shape that is easy for the operator to handle. In addition, the installation stability of the second container 82 on the first container 81 can be improved. The first container 60 shown in FIG. 4 or the first container 70 shown in FIG. 5 may be used instead of the first container 81.

### [Third Embodiment]

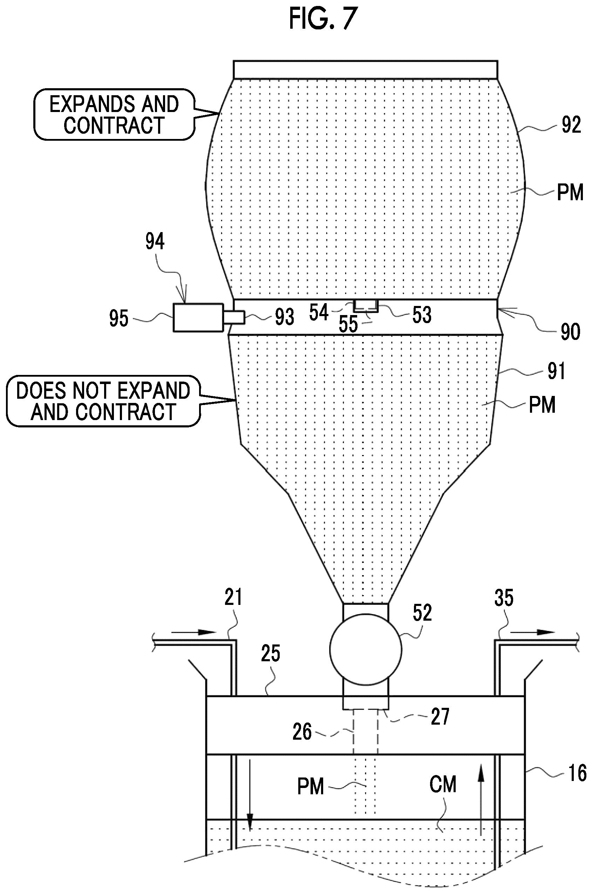

An aspect opposite to the second embodiment may be adopted. That is, as a powdered culture medium supply device 90 shown in FIG. 7 as an example, an aspect in which a first container 91 is formed of a hard resin suitable for USP Class 6 and does not expand and contract and a second container 92 is formed of a soft resin suitable for USP Class 6 and expands and contracts may be adopted.

The first container 91 has a first ventilation port 93 on an upper portion where the powder culture medium PM is not stored. The first ventilation port 93 functions as an exhaust port that discharges air in the first container 91 to the outside in order to eliminate the back pressure in the first container 91 generated in conjunction with the supply of the powder culture medium PM from the second container 92 to the first container 91. In addition, the first ventilation port 93 functions as an intake port that introduces outside air into the first container 91 in order to eliminate the negative pressure in the first container 91 generated in conjunction with the supply of the powder culture medium PM from the first container 91 to the mixing container 16. As in the second ventilation port 83, a filter such as an air vent (Aevent)-50 hydrophobic disposable filter manufactured by Merck KGaA may be attached to the first ventilation port 93.

A desiccant cartridge 95 is exchangeably connected to the first ventilation port 93 as a humidity control mechanism 94. The desiccant cartridge 95 contains, for example, silica gel as a desiccant. The desiccant cartridge 95 dehumidifies the outside air introduced into the first container 91 and sets the relative humidity of the outside air to be equal to or less than a set humidity (for example, 60%). Therefore, a humidity-controlled air is supplied to the inside of the first container 91 from the first ventilation port 93. An indicator that notifies of an exchange period is provided in the desiccant cartridge 95. The desiccant cartridge 95 in which the exchange period has arrived is exchanged by the operator.

As described above, in the third embodiment, the first container 91 has the first ventilation port 93, and the second container 92 expands and contracts. Then, a humidity-controlled air by the humidity control mechanism 94 (desiccant cartridge 95) is supplied to the inside of the first container 91 from the first ventilation port 93. With such a configuration, the supply of the powder culture medium PM from the second container 92 to the first container 91 and the supply of the powder culture medium PM from the first container 91 to the mixing container 16 can be smoothly performed without being hindered by the back pressure and the negative pressure in the first container 91 and the negative pressure in the second container 92. In addition, since the powder culture medium PM in the first container 91 is not likely to solidify due to moisture absorption, the supply of the powder culture medium PM from the first container 91 to the mixing container 16 can be smoothly performed without being hindered by the solidification of the powder culture medium PM in the first container 91. However, since the first container 91 needs to have the first ventilation port 93 and the humidity control mechanism 94 needs to be provided, the powdered culture medium supply device 90 of the third embodiment has a more complicated configuration than the powdered culture medium supply device 17 of the first embodiment and the powdered culture medium supply device 80 of the second embodiment.

The first container 91 does not expand and contract. Therefore, the installation stability of the first container 91 on the feeder 52 and the installation stability of the second container 92 on the first container 91 can be improved.

The first ventilation port that functions as an exhaust port that discharges the air in the first container 91 to the outside in order to eliminate the back pressure in the first container 91 and the first ventilation port that functions as an intake port that introduces the outside air into the first container 91 in order to eliminate the negative pressure in the first container 91 may be separately provided.

### (Modification Example of Third Embodiment)

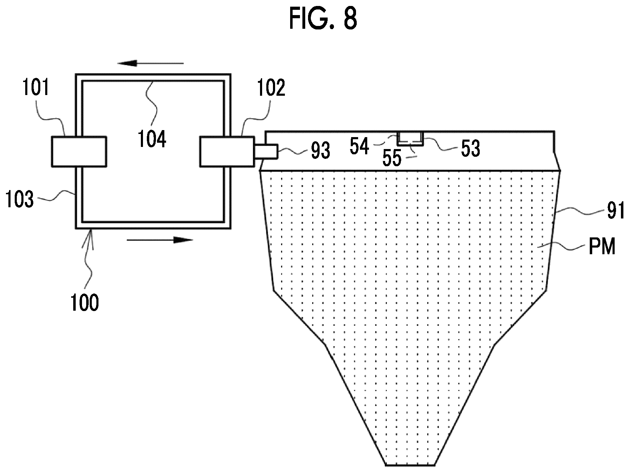

The humidity control mechanism is not limited to the example of the desiccant cartridge 95. As an example, a humidity control mechanism 100 shown in FIG. 8 may be adopted. The humidity control mechanism 100 is composed of a humidity control device 101, a chamber 102 connected to the first ventilation port 93, and a supply line 103 and a return flow path 104 that connect the humidity control device 101 and the chamber 102. The humidity control device 101 is driven and controlled by the control unit 19. The humidity control device 101 takes in outside air. In addition, the humidity control device 101 takes in the air in the first container 91 through the first ventilation port 93, the chamber 102, and the return flow path 104. Then, the relative humidity of the taken-in outside air and the air in the first container 91 is dehumidified to be equal to or less than the set humidity. The humidity control device 101 supplies the outside air and the air dehumidified to the set humidity or less to the first container 91 through the supply line 103, the chamber 102, and the first ventilation port 93.

A pressure gauge is provided in the chamber 102. The control unit 19 determines whether or not a negative pressure or a back pressure is generated in the first container 91 based on the pressure measured by the pressure gauge. In a case in which it is determined that the negative pressure is generated in the first container 91, the control unit 19 drives the humidity control device 101 to supply the outside air and the air in the first container 91 to the first container 91 after dehumidifying the outside air and the air in the first container 91 to the set humidity or less. On the other hand, in a case in which it is determined that the back pressure is generated in the first container 91, the control unit 19 does not drive the humidity control device 101 and discharges the air in the first container 91 to the outside through the first ventilation port 93, the chamber 102, the return flow path 104, and the humidity control device 101. With such a configuration, since the powder culture medium PM in the first container 91 is not likely to solidify due to moisture absorption, the supply of the powder culture medium PM from the first container 91 to the mixing container 16 can be smoothly performed without being hindered by the solidification of the powder culture medium PM in the first container 91.

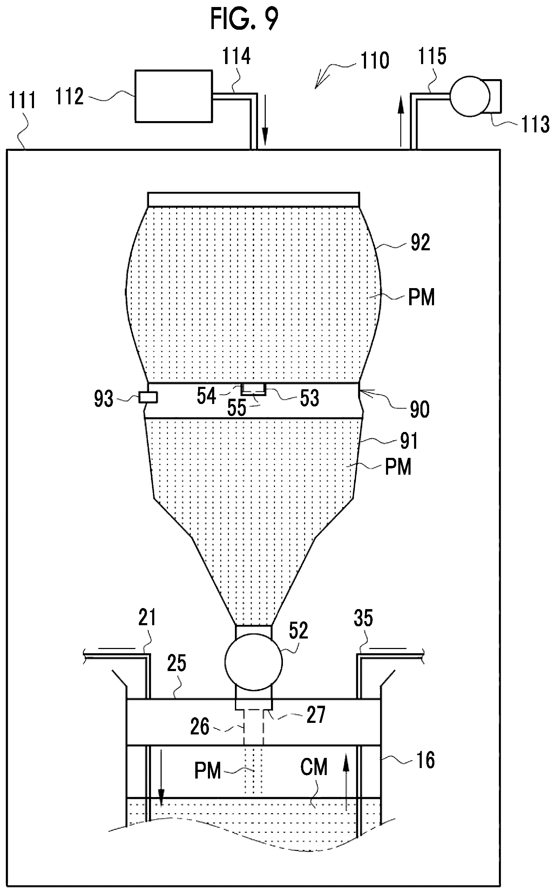

In addition, as an example, a humidity control mechanism 110 shown in FIG. 9 may be adopted. The humidity control mechanism 110 comprises a humidity control room 111, an air handling unit (AHU) 112, and an exhaust fan 113. The humidity control room 111 airtightly covers the entire culture medium preparation device including the mixing container 16 and the powdered culture medium supply device 90. The air conditioner 112 and the exhaust fan 113 are driven and controlled by the control unit 19. The air conditioner 112 takes in outside air and dehumidifies the taken-in outside air to the set humidity or less. The outside air dehumidified to the set humidity or less by the air conditioner 112 is supplied to the humidity control room 111 through a supply line 114. The exhaust fan 113 discharges the air in the humidity control room 111 to the outside through an exhaust path 115. With such a configuration, since the powder culture medium PM in the first container 91 is not likely to solidify due to moisture absorption, the supply of the powder culture medium PM from the first container 91 to the mixing container 16 can be smoothly performed without being hindered by the solidification of the powder culture medium PM in the first container 91. In this case, the operator performs work such as exchanging the second container 92 in the humidity control room 111.

### [Fourth Embodiment]

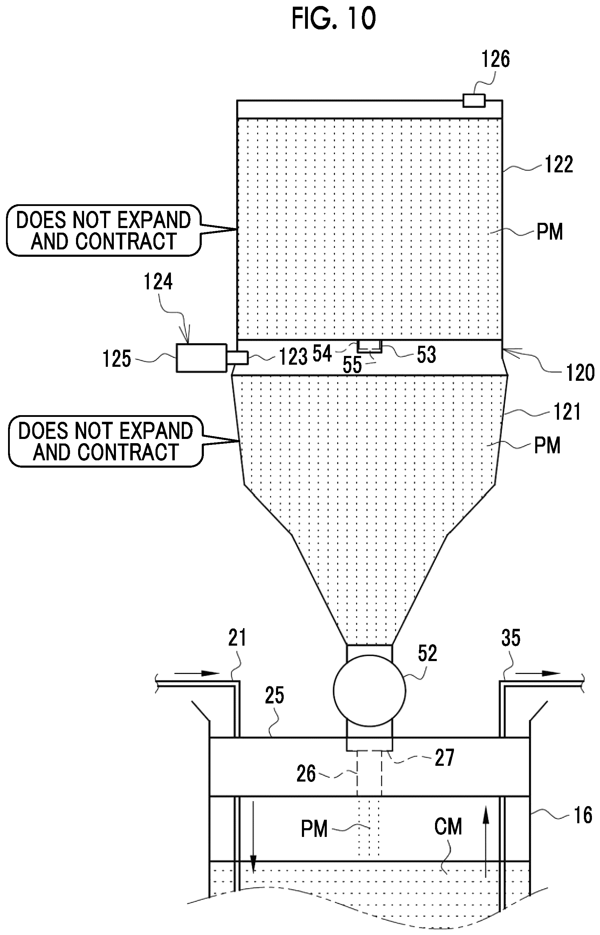

As a powdered culture medium supply device 120 shown in FIG. 10 as an example, an aspect in which both a first container 121 and a second container 122 are formed of a hard resin suitable for USP Class 6 and do not expand and contract may be adopted.

The first container 121 has a first ventilation port 123 on an upper portion where the powder culture medium PM is not stored. The first ventilation port 123 has the same function as the first ventilation port 93 of the third embodiment.

A desiccant cartridge 125 is exchangeably connected to the first ventilation port 123 as a humidity control mechanism 124. The desiccant cartridge 125 has the same function as the desiccant cartridge 95 of the third embodiment.

The second container 122 has a second ventilation port 126 on an upper portion where the powder culture medium PM is not stored. The second ventilation port 126 has the same function as the second ventilation port 83 of the second embodiment.

As described above, in the fourth embodiment, the first container 121 has the first ventilation port 123, and the second container 122 has the second ventilation port 126. Then, a humidity-controlled air by the humidity control mechanism 124 (desiccant cartridge 125) is supplied to the inside of the first container 121 from the first ventilation port 123. With such a configuration, the supply of the powder culture medium PM from the second container 122 to the first container 121 and the supply of the powder culture medium PM from the first container 121 to the mixing container 16 can be smoothly performed without being hindered by the back pressure and the negative pressure in the first container 121 and the negative pressure in the second container 122. In addition, since the powder culture medium PM in the first container 121 is not likely to solidify due to moisture absorption, the supply of the powder culture medium PM from the first container 121 to the mixing container 16 can be smoothly performed without being hindered by the solidification of the powder culture medium PM in the first container 121. However, since the first container 121 needs to have the first ventilation port 123, the second container 122 needs to have the second ventilation port 126, and the humidity control mechanism 124 needs to be provided, the powdered culture medium supply device 120 of the fourth embodiment has a more complicated configuration than the powdered culture medium supply device 17 of the first embodiment, the powdered culture medium supply device 80 of the second embodiment, and the powdered culture medium supply device 90 of the third embodiment.

As the humidity control mechanism, the humidity control mechanism 100 shown in FIG. 8 or the humidity control mechanism 110 shown in FIG. 9 may be used instead of the humidity control mechanism 124 composed of the example of the desiccant cartridge 125.

As in the second container 82 of the second embodiment or the second container 122 of the fourth embodiment, in a case in which the second ventilation port 83 or 126 is provided, a humidity control mechanism that dehumidifies the air introduced into the second container 82 or 122 through the second ventilation port 83 or 126 to the set humidity or less may be provided. By preventing the powder culture medium PM in the second container 82 or 122 from absorbing moisture in this way, the powder culture medium PM can be smoothly supplied from the second container 82 or 122 to the first container 81 or 121. In addition, in a case in which the powder culture medium PM in the second container 82 or 122 is prevented from absorbing moisture, the solidification of the powder culture medium PM supplied to the first container 81 or 121 due to moisture absorption can also be effectively prevented. As a result, it is possible to contribute to the smooth supply of the powder culture medium PM from the first container 81 or 121 to the mixing container 16, which is more preferable.

### Examples

First, the equipment used in the examples will be described. A beaker (model number: B-200 SCI, capacity: 200 mL) manufactured by HARIO Co., Ltd. was used as the mixing container 16. As the feeder 52, a micron feeder (model number TF-70-CT (AD)) manufactured by Aisin Nano Technology Co., Ltd. was used.

For each flow passage such as the pure water supply line 21, the culture medium supply line 35, and the waste liquid line 42, a lab silicone tube (product number: 9-869-07, inner diameter × outer diameter = φ4 mm × φ6 mm) manufactured by AS ONE Corporation or the like was used after being appropriately cut.

A Masterflex pump (model number: 07528-30) manufactured by Yamato Scientific Co., Ltd. was used as a driving unit of the pure water supply pump 22 and the culture medium supply pump 36. A Masterflex pump head (model number: 77201-60) manufactured by Yamato Scientific Co., Ltd. was used as a pump head of the pure water supply pump 22 and the culture medium supply pump 36. In the driving units of the pure water supply pump 22 and the culture medium supply pump 36, the above-described Masterflex pump was shared for both purposes, and two of the above-described Masterflex pump heads were connected in tandem to one Masterflex pump.

A clamp-on type flow sensor (model number: FD-XA1) manufactured by KEYENCE CORPORATION was used as the pure water flowmeter 23 and the culture medium flowmeter 37.

An Ultra stirrer (model number: MSD-1) manufactured by AS ONE Corporation was used as the stirrer 32. As the stirring bar 33, a regular stirrer bar (model number: C 8 × 30, diameter × length = φ8 mm × 30 mm) manufactured by AS ONE Corporation was used.

As the main bodies of the conductivity meter 39 and the hydrogen-ion exponent meter 40, a benchtop potential hydrogen (pH) and conductivity meter (model number: F-74) manufactured by Horiba, Ltd. was also shared for both purposes. As the electrode of the conductivity meter 39, a general-purpose conductivity cell (model number: 3562-10D) manufactured by HORIBA, Ltd. was used. As an electrode of the hydrogen-ion exponent meter 40, a non-refillable pH electrode (model number: 9652-20D) manufactured by HORIBA, Ltd. was used. In all of these electrodes, the conductivity and the hydrogen-ion exponent can be measured inline. As the flow cell installed together with these electrodes, a flow cell (product number: 3200844642) manufactured by HORIBA, Ltd. was used.

As the first container and the second container that expand and contract, such as the first container 50 and the second container 51, the first container 81, and the second container 92, a charge bag (model number: PE-S) manufactured by Charge Point, Inc. was used. In addition, as the first container and the second container that do not expand and contract, such as the second container 82, the first container 91, and the first container 121 and the second container 122, a hard resin processed into a tubular shape was used.

As the valve 55, a single-use split butterfly valve passive (model number: SUP) manufactured by Charge Point, Inc. was used. The humidity control mechanism 100 shown in FIG. 8 was adopted as the humidity control mechanism. As the humidity control device 101, a humidity adjustment unit (model number: STU-1) manufactured by AS ONE Corporation was used.

As the powder culture medium PM, BalanCD (R) CHO Growth A manufactured by FUJIFILM Irvine Scientific, Inc. was used.

Next, the experimental conditions of the examples will be described. The flow rate of the pure water PW supplied to the mixing container 16 is 80 mL/min. The supply amount of the powdered culture medium PM to the mixing container 16 per unit time is 0.76 g/min. The rotation speed of the screw of the feeder 52 is 1 rotation per minute (rpm). The amount of the liquid in the mixing container 16 is 100 mL. The rotation speed of the stirrer 32 is 300 rpm. The measurement interval of the conductivity meter 39 and the hydrogen-ion exponent meter 40 is 10 seconds.

The flow rate of the pure water PW was calculated from the supply amount of the powdered culture medium PM, with a value that matched the "amount of water to be put in with respect to a certain amount of powder" described in the instruction manual of the powdered culture medium PM. As the supply amount of the powdered culture medium PM, a value obtained by actually supplying the powdered culture medium PM by rotating the screw of the feeder 52 at a set rotation speed and measuring the weight of the powdered culture medium PM supplied in a unit time with an electronic balance was used. As the electronic balance, a compact balance (model number: EW-150i) manufactured by A&D Company, Limited was used.

As described above, since the supply amount of the pure water PW to the mixing container 16 and the discharge amount of the culture medium CM from the mixing container 16 was appropriately controlled, the amount of the liquid in the mixing container 16 did not change from the beginning to the end. In a case where the amount of the liquid in the mixing container 16 before the continuous supply of the pure water PW and the powdered culture medium PM was started, that is, the amount of the initial solvent was too large, the time taken for the concentration of the culture medium CM to reach the set concentration was prolonged, or partial bias occurred in the concentration of the culture medium CM in the mixing container 16. On the other hand, in a case where the amount of the initial solvent in the mixing container 16 before the continuous supply of the pure water PW and the powdered culture medium PM was started was too small, the supply amount of the powdered culture medium PM became too large with respect to the amount of the initial solvent, and poor dissolution tended to occur, for example, in a case where the powdered culture medium PM accumulated on the liquid surface. In consideration of this, the amount of the liquid in the mixing container 16 was set to 100 mL, which was neither too much nor too little with respect to the supply amount of the powdered culture medium PM.

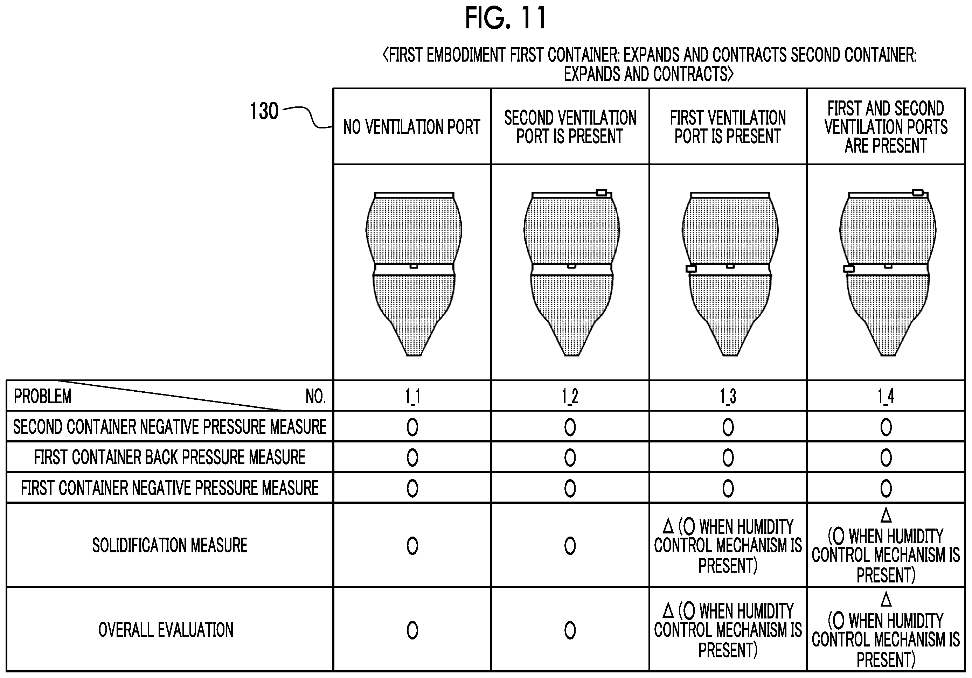

Using the above-described equipment and conditions, the combination that can be considered in the first to fourth embodiments was actually evaluated for whether or not the powder culture medium PM was supplied from the second container to the first container and the powder culture medium PM was supplied from the first container to the mixing container, and whether or not the measures against the four problems described above were taken. The evaluation results are shown in Tables 130 to 133 of FIGS. 11 to 14.

Here, the measures against the four problems are measures against the negative pressure in the second container (hereinafter, referred to as a second container negative pressure measure), measures against the back pressure in the first container (hereinafter, referred to as a first container back pressure measure), measures against the negative pressure in the first container (hereinafter, referred to as a first container negative pressure measure), and measures against the solidification of the powder culture medium PM in the first container due to moisture absorption (hereinafter, referred to as a solidification measure).

In Tables 130 to 133, a circle mark (○) is given in a case in which the measures against the problems are taken and the problems are overcome, and a triangle mark (△) is given in a case in which the measures against the problems are not taken and the problems are not overcome. In the overall evaluation, a circle mark (○) is given in a case in which all of the measures against the four problems are taken and all of the four problems are overcome, and a triangle mark (△) is given in a case in which at least one of the measures against the four problems is not taken and at least one of the four problems is not overcome.

The second container negative pressure measure is ○ in a case in which the powder culture medium PM can be smoothly supplied from the second container to the first container without the inside of the second container being in a negative pressure. On the other hand, the second container negative pressure measure is △ in a case in which the inside of the second container is in a negative pressure and the supply of the powder culture medium PM from the second container to the first container is delayed.

Similarly, the first container back pressure measure is ○ in a case in which the powder culture medium PM can be smoothly supplied from the second container to the first container without the back pressure being generated in the first container. On the other hand, the first container back pressure measure is △ in a case in which the back pressure is generated in the first container and the supply of the powder culture medium PM from the second container to the first container is delayed.

In addition, the first container negative pressure measure is ○ in a case in which the powder culture medium PM can be smoothly supplied from the first container to the mixing container 16 without the inside of the first container being in a negative pressure. On the other hand, the first container negative pressure measure is △ in a case in which the inside of the first container is in a negative pressure and the supply of the powder culture medium PM from the first container to the mixing container 16 is delayed.

The solidification measure is ○ in a case in which the powder culture medium PM in the first container does not solidify due to moisture absorption and the powder culture medium PM can be smoothly supplied from the first container to the mixing container 16. On the other hand, the solidification measure is △ in a case in which the powder culture medium PM in the first container solidifies due to moisture absorption and the supply of the powder culture medium PM from the first container to the mixing container 16 is delayed.

Table 130 shown in FIG. 11 summarizes the combination that can be considered in the first embodiment in which both the first container and the second container expand and contract and the evaluation thereof. No. 1_1 is a configuration in which the first container and the second container are not provided with the ventilation ports (the first container and the second container are sealed), which is shown in FIG. 2. In this case, all of the problems are overcome, so that the overall evaluation is ○. No. 1_2 is a configuration in which the first container is sealed and the second container is provided with the second ventilation port. In this case as well, all of the problems are overcome, so that the overall evaluation is ○.

No. 1_3 is a configuration in which the first container is provided with the first ventilation port and the second container is sealed, and No. 1_4 is a configuration in which the first container is provided with the first ventilation port and the second container is provided with the second ventilation port. In these cases, the overall evaluation is ○ on the condition that the solidification measure is taken by the humidity control mechanism.

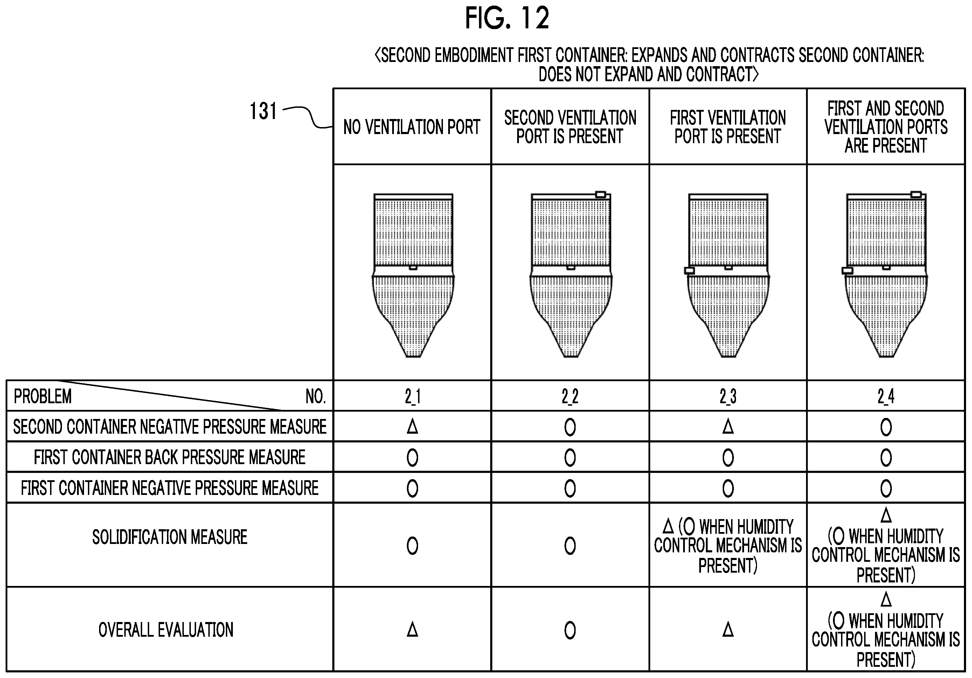

Table 131 shown in FIG. 12 summarizes the combination that can be considered in the second embodiment in which the first container expands and contracts and the second container does not expand and contract and the evaluation thereof. No. 2_1 is a configuration in which the first container and the second container are not provided with the ventilation ports (the first container and the second container are sealed). In this case, since the second container negative pressure measure is not taken, the overall evaluation is △. No. 2_2 is a configuration in which the first container is sealed and the second container is provided with the second ventilation port, which is shown in FIG. 6. In this case, all of the problems are overcome, so that the overall evaluation is ○.

No. 2_3 is a configuration in which the first container is provided with the first ventilation port and the second container is sealed, and No. 2_4 is a configuration in which the first container is provided with the first ventilation port and the second container is provided with the second ventilation port. In the case of No. 2_3, since the second container negative pressure measure is not taken as in the case of No. 2_1, the overall evaluation is △. In the case of No. 2_4, the overall evaluation is ○ on the condition that the solidification measure is taken by the humidity control mechanism.

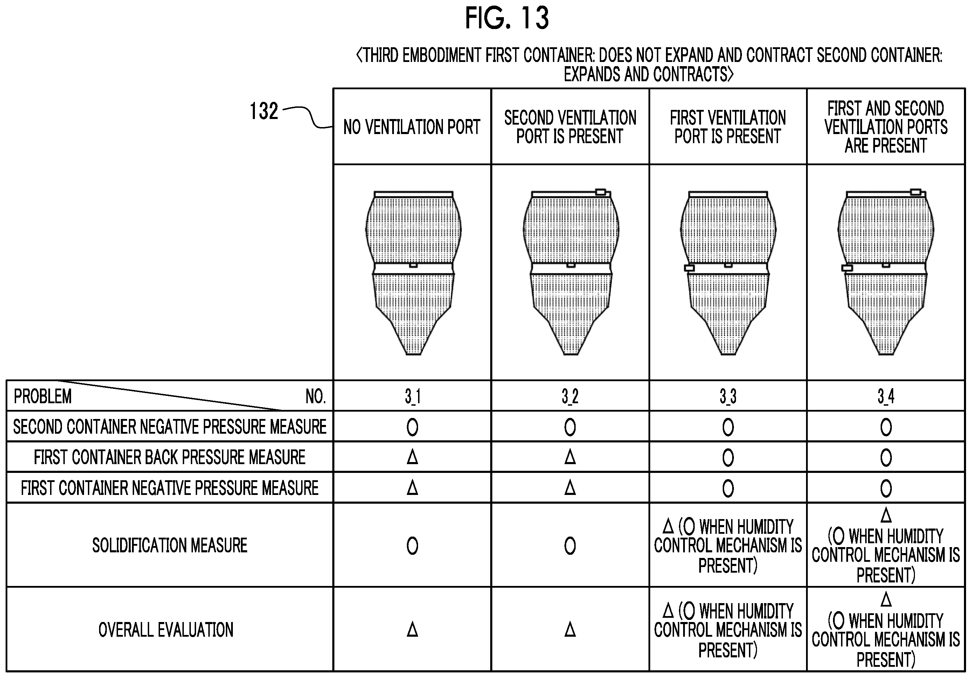

Table 132 shown in FIG. 13 summarizes the combination that can be considered in the third embodiment in which the first container does not expand and contract and the second container expands and contracts and the evaluation thereof. No. 3_1 is a configuration in which the first container and the second container are not provided with the ventilation ports (the first container and the second container are sealed). In addition, No. 3_2 is a configuration in which the first container is sealed and the second container is provided with the second ventilation port. In these cases, since the first container back pressure measure and the first container negative pressure measure are not taken, the overall evaluations are both △.

No. 3_3 is a configuration in which the first container is provided with the first ventilation port and the second container is sealed, which is shown in FIG. 7, and No. 3_4 is a configuration in which the first container is provided with the first ventilation port and the second container is provided with the second ventilation port. In these cases, the overall evaluation is ○ on the condition that the solidification measure is taken by the humidity control mechanism.

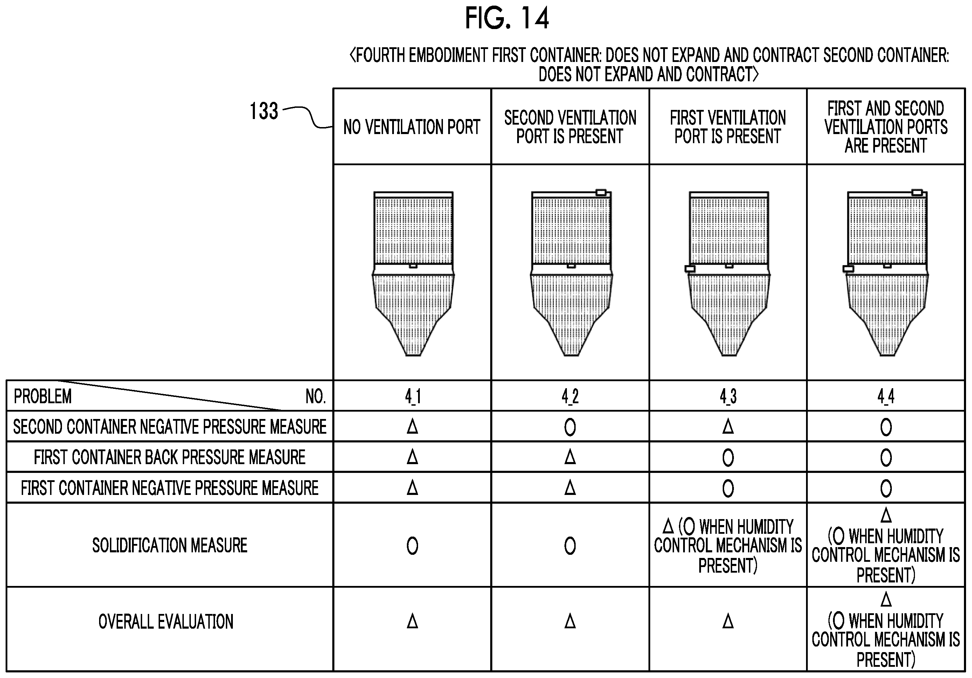

Table 133 shown in FIG. 14 summarizes the combination that can be considered in the fourth embodiment in which both the first container and the second container do not expand and contract and the evaluation thereof. No. 4_1 is a configuration in which the first container and the second container are not provided with the ventilation ports (the first container and the second container are sealed). In this case, since the measures other than the solidification measure are not taken, the overall evaluation is △. No. 4_2 is a configuration in which the first container is sealed and the second container is provided with the second ventilation port. In this case, since the first container back pressure measure and the first container negative pressure measure are not taken, the overall evaluation is △.

No. 4_3 is a configuration in which the first container is provided with the first ventilation port and the second container is sealed, and No. 4_4 is a configuration in which the first container is provided with the first ventilation port and the second container is provided with the second ventilation port, which is shown in FIG. 10. In the case of No. 4_3, since the second container negative pressure measure is not taken, the overall evaluation is △. In the case of No. 4_4, the overall evaluation is ○ on the condition that the solidification measure is taken by the humidity control mechanism.

As described above, it was confirmed that, according to the aspects shown in the first to fourth embodiments (No. 1_1 of FIG. 11, No. 2_2 of FIG. 12, No. 3_3 of FIG. 13, and No. 4_4 of FIG. 14), all of the problems can be overcome, although there is a condition that the solidification measure is taken by the humidity control mechanism.

Since the powder culture medium PM has a relatively high bulk density and is not easily permeable to air, a negative pressure and a back pressure are likely to be generated in the first container and/or the second container. In addition, the powder culture medium PM has a relatively high hygroscopicity and is likely to solidify. Therefore, in a case in which the powder culture medium PM is used as the powder, the effect that all of the problems can be overcome is more exhibited.

The sensor may detect that the amount of the powder culture medium PM in the first container is equal to or less than the set amount, and the valve 55 may be automatically opened without the operator. Similarly, the sensor may detect that the supply of the powder culture medium PM from the second container to the first container is ended, and the valve 55 may be automatically closed without the operator.

The weight of the first container may be measured by the weight meter instead of or in addition to the weight of the mixing container 16 being measured by the weight meter. Then, the amount of the powder culture medium PM supplied from the first container to the mixing container 16 may be controlled such that the amount of decrease in the measured weight per unit time is constant. In this case, it is preferable to temporarily stop the control while the second container is being exchanged and/or while the powder culture medium PM is being supplied from the second container to the first container. Alternatively, the control may be continued based on the measured weight immediately before the second container is exchanged and/or immediately before the powder culture medium PM is supplied from the second container to the first container while the second container is being exchanged and/or while the powder culture medium PM is being supplied from the second container to the first container. In a case in which the control is continued based on the immediately preceding measured weight, the measured weight until then is reset after the second container is exchanged and/or after the powder culture medium PM is supplied from the second container to the first container, the weight is newly measured, and the control is resumed based on the newly measured weight.

In a case in which the second container is connected to the first container, the vibration of the second container is transmitted to the first container, and the measured weight of the first container is likely to be unstable. Therefore, it is preferable to connect the first container and the second container by a method in which the vibration of the second container is not transmitted to the first container. Specifically, it is preferable that the connection between the first container and the second container is flexible fixation instead of rigid fixation. For example, it is preferable that a member (hook or the like) that supports the weight of the first container and a member (hook or the like) that supports the weight of the second container are independent of each other.

The conductivity measured by the conductivity meter 39 is a representative value obtained by summing the conductivities of the respective components dissolved in the culture medium CM. Therefore, to more accurately determine whether or not the concentration of the culture medium CM has reached the set concentration, it is preferable to measure the concentration of each component of the culture medium CM. However, since it is not easy to measure the concentration of each component of the culture medium CM, a representative value of the conductivity of each component is measured instead.

In a case where the concentration of each component of the culture medium CM can be measured, it is best to determine, based on the result, whether or not the concentration of the culture medium CM has reached the set concentration. Provided that in a case where the concentration of each component of the culture medium CM is measured, there is a possibility that the time taken for each component to reach the set concentration may vary. Therefore, in a case where it is determined that all the components have reached the set concentration, it is necessary to stop discarding the culture medium CM and start supplying the culture medium CM to the culture tank 11.

The characteristics of the culture medium CM to be supplied to the culture tank 11, which are to be measured inline, are not limited to the exemplified conductivity and hydrogen-ion exponent. In addition to or instead of these, a Raman spectrum, an infrared absorption spectrum, a near infrared absorption spectrum, an ultraviolet absorption spectrum, a fluorescence spectrum, and the like may be measured. Then, the concentration of the culture medium CM may be derived based on the measurement results of these spectra.

In each of the embodiments, pure water PW is exemplified as the solvent and the purified water, but the present invention is not limited thereto. Distilled water, water for injection (WFI), or the like may be used. In addition, in each of the embodiments, the culture medium CM is exemplified as the bioprocess solution, but the present invention is not limited thereto. The bioprocess solution may be a buffer solution. Therefore, the powder is not limited to the example of the powder culture medium, and may be a powder constituting a solid component of the buffer solution.

The technology according to the following supplementary notes can be understood based on the above description.

### [Supplementary Note 1]

A powder supply device that continuously supplies a powder in order to continuously prepare a bioprocess solution having a set concentration in a mixing container, the powder supply device comprising:
a first container that stores the powder, the first container being connected to the mixing container via a feeder and supplying the powder to the feeder; and
a second container that stores the powder, the second container being connected to the first container and supplying the powder to the first container.

### [Supplementary Note 2]

The powder supply device according to Supplementary Note 1, in which the second container is exchangeably connected to the first container.

### [Supplementary Note 3]

The powder supply device according to Supplementary Note 1 or 2, in which the first container expands and contracts.

### [Supplementary Note 4]

The powder supply device according to Supplementary Note 3, in which the first container is sealed.

### [Supplementary Note 5]

The powder supply device according to Supplementary Note 3 or 4, in which a part of the first container expands and contracts.

### [Supplementary Note 6]

The powder supply device according to any one of Supplementary Notes 1 to 5, in which the second container expands and contracts.

### [Supplementary Note 7]

The powder supply device according to Supplementary Note 6, in which the second container is sealed.

### [Supplementary Note 8]

The powder supply device according to Supplementary Note 6 or 7, in which a part of the second container expands and contracts.

### [Supplementary Note 9]

The powder supply device according to any one of Supplementary Notes 1 to 8, in which the first container has a first ventilation port.

### [Supplementary Note 10]

The powder supply device according to Supplementary Note 9, in which a humidity-controlled air is supplied to an inside of the first container from the first ventilation port by a humidity control mechanism.

### [Supplementary Note 11]

The powder supply device according to Supplementary Note 9 or 10, in which the first container does not expand and contract.

### [Supplementary Note 12]

The powder supply device according to any one of Supplementary Notes 1 to 11, in which the second container has a second ventilation port.

### [Supplementary Note 13]

The powder supply device according to Supplementary Note 12, in which the second container does not expand and contract.

### [Supplementary Note 14]

The powder supply device according to any one of Supplementary Notes 1 to 13, in which the first container and the second container expand and contract.

### [Supplementary Note 15]

The powder supply device according to any one of Supplementary Notes 1 to 13,
in which the first container expands and contracts, and
the second container has a second ventilation port.

### [Supplementary Note 16]

The powder supply device according to any one of Supplementary Notes 1 to 13,
in which the first container has a first ventilation port,
the second container expands and contracts, and
a humidity-controlled air is supplied to an inside of the first container from the first ventilation port by a humidity control mechanism.

### [Supplementary Note 17]

The powder supply device according to any one of Supplementary Notes 1 to 13,
in which the first container has a first ventilation port,
the second container has a second ventilation port, and
a humidity-controlled air is supplied to an inside of the first container from the first ventilation port by a humidity control mechanism.

### [Supplementary Note 18]

The powder supply device according to any one of Supplementary Notes 1 to 17,
in which a solvent that is mixed with the powder in the mixing container is purified water, the powder being a powder culture medium, and
the bioprocess solution is a culture medium.

### [Supplementary Note 19]

The powder supply device according to Supplementary Note 18,
in which the culture medium is continuously supplied from the mixing container to a culture tank.

### [Supplementary Note 20]

The powder supply device according to any one of Supplementary Notes 1 to 19,
in which the first container and the second container are used once and then discarded.

The technology of the present disclosure can also be combined with various embodiments and/or various modification examples described above, as appropriate. Additionally, the technology of the present disclosure is not limited to each of the above-described embodiments, and various configurations can, of course, be employed without departing from the gist.

The above-described contents and the above-shown contents are the detailed description of the parts according to the technology of the present disclosure, and are merely an example of the technology of the present disclosure. For example, the above description of the configuration, the function, the operation, and the effect are the description of examples of the configuration, the function, the operation, and the effect of the parts according to the technology of the present disclosure. Accordingly, it goes without saying that unnecessary parts may be deleted, new elements may be added, or replacements may be made with respect to the above-described contents and the above-shown contents within a range that does not deviate from the gist of the technology of the present disclosure. Moreover, in order to avoid complications and facilitate grasping the parts according to the technology of the present disclosure, in the above-described contents and the above-shown contents, the description of technical general knowledge and the like that do not particularly require description for enabling the implementation of the technology of the present disclosure are omitted.

In the present specification, "A and/or B" has the same meaning as "at least one of A or B". That is, the term "A and/or B" means only A, only B, or a combination of A and B. In addition, the bonding was bonded such that

Further, in the present specification, in a case where three or more items are expressed in combination using "and/or", the same concept as that of "A and/or B" applies.

All of the documents, the patent applications, and the technical standards described in the present specification are incorporated herein by reference to the same extent as in a case in which each of the documents, patent applications, and technical standards is specifically and individually described by being incorporated by reference.

## Claims

1. A powder supply device that continuously supplies a powder in order to continuously prepare a bioprocess solution having a set concentration in a mixing container, the powder supply device comprising:
a first container that stores the powder, the first container being connected to the mixing container via a feeder and supplying the powder to the feeder; and
a second container that stores the powder, the second container being connected to the first container and supplying the powder to the first container.

2. The powder supply device according to claim 1,
wherein the second container is exchangeably connected to the first container.

3. The powder supply device according to claim 1,
wherein the first container expands and contracts.

4. The powder supply device according to claim 3,
wherein the first container is sealed.

5. The powder supply device according to claim 3,
wherein a part of the first container expands and contracts.

6. The powder supply device according to claim 1,
wherein the second container expands and contracts.

7. The powder supply device according to claim 6,
wherein the second container is sealed.

8. The powder supply device according to claim 6,
wherein a part of the second container expands and contracts.

9. The powder supply device according to claim 1,
wherein the first container has a first ventilation port.

10. The powder supply device according to claim 9,
wherein a humidity-controlled air is supplied to an inside of the first container from the first ventilation port by a humidity control mechanism.

11. The powder supply device according to claim 9,
wherein the first container does not expand and contract.

12. The powder supply device according to claim 1,
wherein the second container has a second ventilation port.

13. The powder supply device according to claim 12,
wherein the second container does not expand and contract.

14. The powder supply device according to claim 1,
wherein the first container and the second container expand and contract.

15. The powder supply device according to claim 1,
wherein the first container expands and contracts, and
the second container has a second ventilation port.

16. The powder supply device according to claim 1,
wherein the first container has a first ventilation port,
the second container expands and contracts, and
a humidity-controlled air is supplied to an inside of the first container from the first ventilation port by a humidity control mechanism.

17. The powder supply device according to claim 1,
wherein the first container has a first ventilation port,
the second container has a second ventilation port, and
a humidity-controlled air is supplied to an inside of the first container from the first ventilation port by a humidity control mechanism.

18. The powder supply device according to claim 1,
wherein a solvent that is mixed with the powder in the mixing container is purified water, the powder being a powder culture medium, and
the bioprocess solution is a culture medium.

19. The powder supply device according to claim 18,
wherein the culture medium is continuously supplied from the mixing container to a culture tank.

20. The powder supply device according to claim 1,
wherein the first container and the second container are used once and then discarded.
